# EUROPEAN PATENT APPLICATION

(11) **EP 1 992 345 A1**
(43) Date of publication of application: **19.11.2008**
(21) Application number: 06783813.6
(22) Date of filing: 31.07.2006
(51) Int. Cl.: A61K 31/426, A61K 31/695, A61K 33/06, A61P 1/14

(54) **SYNERGIC COMBINATION OF H2-RECEPTOR INHIBITORS, INERT SILICONE AND A HYDROXYMAGNESIUM ALUMINATE COMPLEX**

(30) Priority: 09.03.2006 MX PA06002749
(71) Applicant: ESPINOZA ABDALA, Leopoldo de Jesús, Jalisco Mexico (MX); WORLD-TRADE IMPORT-EXPORT, WTIE, A.G., 6320 Zug (CH)
(72) Inventor: GARCÍA ARMENTA, María Elena, C.P. 45020 Guadalajara, Jalisco (MX); ALVAREZ OCHOA, Victor Guillermo, C.P. 45222 Zapopan, Jalisco, México (MX); SANTOS MURILLO, Josefina, C.P. 44600 Zapopan, Jalisco (MX); GARCIA ARMENTA, Patricia, C.P. 45110 Zapopan, Jalisco, México (MX)
(74) Representative: Carvajal y Urquijo, Isabel
(86) International application number: PCT/MX2006/000084
(87) International publication number: WO 2007/102726

(57) **Abstract**

The present invention relates to the pharmaceutical industry in general and to the pharmaceutical industry that prepares medicinal products for the prevention and/or treatment of peptic acid disorders and symptoms related to the excess production of gastric acid, in addition to contributing to cicatrization and the prevention of recurrences of symptoms and complications, in particular. It has advantages as compared with prior-art compositions, such as a smaller dose of each element to prevent or substantially to reduce the secondary effects and a therapeutic effect at least equal to the effects of prior-art compositions. This composition is **characterized in that** it comprises a type-2 histamine-receptor-inhibiting agent, an antiflatulence agent, an antacid, and also pharmaceutically acceptable excipients.

## Description

### FIELD OF THE INVENTION

The present invention generally relates to pharmaceutical industry and, in particular, to drug preparer pharmaceutical industry for prevention and/or treatment of acid-peptic disorders and symptoms related to excess production of gastric acid in addition to contributing to the cicatrization, prevention of recurrences of symptoms and complications. More specifically, the invention relates to a combination of H₂-receptor inhibitors such as Famotidine or Ranitidine; a methylated siloxane lineal polymer (inert silicone) such as Dimeticone or Simeticone and an antacid such as aluminum, magnesium or calcium salts, e.g., a hydroxymagnesium aluminate complex such as Magaldrate.

### BACKGROUND OF THE INVENTION

Currently, many people experience or suffer from gastrointestinal disorders ranging from acid-peptic disease, gastritis, colitis to more severe disorders such as gastroduodenal ulcers and cancer in the digestive system. Many of these diseases originate from the individuals' nutrition to other type of etiology such as certain infections, nervous disorders among many other causes.

It should be remembered that the digestive systems begins in the mouth, so that digestion begins in the mouth. The first step for a good digestion is a good mastication; from there, the bolus moves into the esophagus which, through a series of movements called peristaltic movements, moves the bolus into the stomach where it is temporarily stored and mixed with gastric juices to then pass though the pylorus into the duodenum. Pepsin (enzyme which aids in protein digestion) is found in gastric juices and continuous with the digestion of food in addition to practically sterilizing the content in the stomach in order to avoid the breakdown thereof.

The food remains in the stomach for a period from one to five hours depending on its source; carbohydrate-based food such as bread, sugar or potatoes remain less time than protein-based food such as meat. The muscular movements of the stomach allow their content to be well mixed with digestive juices and then to be progressively moved into the outlet of the stomach called pylorus and to pass to the small intestine which performs the main part of the digestion process.

One of the most common diseases from the digestive system is the acid-peptic one (including diseases such as reflux esophagitis, gastroesophageal reflux, hiatal hernia, acute and chronic gastritis, gastric ulcer, duodenal ulcer. Alkaline, ethyl and medicament gastritis, non-ulcerous dyspepsia, stress ulcer), caused by hydrochloric acid hypersecretion of an enzyme called gastrin, whose function is to balance the acidity of the stomach.

Likewise, the deficiency of the gastric mucous membrane protection mechanisms against acid is also a factor for the development of this condition. Acid-peptic disease has a highly prevalence worldwide with a global percentage reaching to 10% and regionally to figures as high as 20 or 30%.

When stomach is overloaded or frequently limited due to an inadequate nutrition, the functions thereof and its whole regulating system become altered which can lead to different diseases such as gastritis, which is an inflammation of the mucous membrane lining the stomach.

There are factors which can cause a chronic gastritis such as excessive eating, emotional stress, fatrich food intake, hot spices, condiments, alcoholic drinks consumption, coffee, nicotinism, infections and lack of certain nutrients and vitamins. When gastritis presents in an acute form, it is often as a consequence of the intake of food or medicaments which irritates the stomach wall. An excessive alcohol consumption, food intoxication, emotional stress and infections also cause gastritis. The symptoms which often come with acute gastritis may persist for some time and develop from an acute gastritis to a chronic gastritis, which can cause a loss of the cells lining the stomach with the consequent reduction of gastric juices secretion.

Under normal conditions, the inner wall from the digestive system is protected by a mucous layer; if this lining becomes thinner or destroyed, an ulcer develops which causes a burning painful sensation. Emotional stress causing an excessive acid secretion; as well as nicotinism, an inadequate nutrition, insomnia, excessive caffeine intake and the use of chemical substances which irritate the stomach wall can exacerbate or cause ulcers. Therefore, it is necessary to avoid the intake of the above-mentioned so that the patient with ulcer is able to limit the gastric secretion and allow the ulcer recovery. Antacids are useful for neutralization of gastric acids; the intake thereof is an hour after the food is eaten. Soft diets are useful in case of relapse. Peptic ulcer is an indication that there is a serious disorder.

The most common clinical manifestations are: pyrosis (burning sensation behind the sternum) which is often confound with cardiovascular diseases; epigastralgia (pain in the pit of the stomach); and dyspepsia or difficulty in the process of digestion, accompanied by bloating, heaviness sensation and presence of gasses.

On the other hand, stress is one of the factors which influence in the development of the acid-peptic disease. Under a situation of stress, the autonomic nervous system discharges a great amount of neurotransmitters which, in turn, cause the secretion of gastrin and hydrochloric acid; along with this, there are bad hygienic-dietary habits which condition the appearance of the illness. Moreover, the natural defense systems from the stomach lose its working capacity, presenting hence the senile patient a higher risk.

Also, there is a bacteria which may be the cause of the acid-peptic disease called Helicobacter pylori, which directly alters the stomach defense mechanisms for protection against acid and, at the same time, increases in *situ* the acid secretion within the organ. This bacteria lives within the stomach and can be directly transmitted by being in contact with the saliva of an infected individual.

Presently, the most suitable treatment for attacking this acid-peptic disease and the consequences thereof is that which wholly controls the appearance of heartburn and ulcers. Antacids which are sold without a medical prescription only soothe the symptoms, but do not relieve the disease.

The indiscriminate use of medicaments known as non-steroidal antiinflammatory drugs such as aspirin, diclofenac, naproxen and ibuprofen increase the appearance of the acid-peptic disease.

Other compounds have already individually been analyzed for treating the charts mentioned above. A presentation is hereinafter made of the compounds which are considered the most promising, though with drawbacks, in treating the charts already mentioned.

Chemically famotidine is the N'-(aminosulfonyl)-3-[[[2-[(diaminomethylene)amine]-4-thiazolyl]methyl]thio]propanimldamide; it is a white powder with the formula C₈H1₅N₇O₂S₃ and corresponds to the following formula:

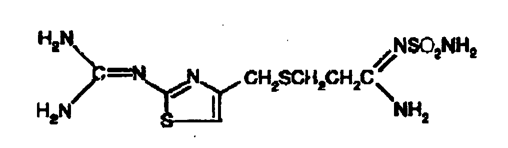

Famotidine competitively inhibits the binding of histamine to the H₂-receptors in the basal membrane of the parietal cells, reducing the stomach acid secretion stimulated by food and other agonists such as caffeine or insulin. Famotidine also reduces the total volume of gastric juice by indirectly inhibiting pepsin secretion. This drug does not alter gastric motility, evacuation of stomach esophageal pressure, and bile or pancreatic secretions. Famotidine increases gastric pH, favoring the cicatrization of the ulcers by protecting the mucous membrane from irritating effects caused by non-steroidal antiinflammatory drugs. Famotidine can be administered orally and parenterally.

On a ponderal basis, Famotidine exerts an antisecreting effect from 20 to 30 times more potent than cimetidine and from 8 to 10 times more potent than ranitidine. Famotidine lacks of affinity toward androgenic receptors. Famotidine has little or no effect on the serum levels of gastrin at fasting or after meals.

The evacuation of the stomach and the exocrine function from the pancreas are not affected by Famotidine. Famotidine is used for treating ulcers (injuries in the inner lining of the stomach or the small intestine); gastroesophageal reflux disease (GERD), a condition where the acid reflux from the stomach causes pyrosis (heartburn) and lesions in the esophagus; and other conditions where the stomach produces too much acid, such as Zollinger-Ellison syndrome (tumors in the pancreas or small intestine which cause an increased production of gastric acid).

Famotidine without a medical prescription is used for preventing and treating the symptoms of pyrosis, associated with acid indigestion and upset stomach, caused by eating or drinking certain food or beverages. As a general rule, famotidine is well tolerated, causing few adverse reactions.

Dimeticone or Simeticone is an antiflatulence and defrothing agent active orally which is used to relieve pain and abdominal troubles caused by the pressure of an excess of gasses. Chemically is a lineal polymer of methylated siloxanes (inert silicone), with a number of units between 200 and 350 depending on its viscosity.

Due to the fact that dimeticone is an inert molecule, it cannot be absorbed since it is not transformed by intestinal flora, so it is expelled through feces. It has surface-active properties which decrease the surface tension of mucogaseous bubbles, responsible for gas retention, thus allowing its disintegration and avoiding its formation, originating a carminative and antiflatulence effect. Interactions of dimeticone with other drugs or herbs have not been communicated. The adverse effects of dimeticone are, in general, mild and transitory. The most frequent adverse effect is moderated constipation.

Magaldrate is an antacid agent made-up of an aluminate-magnesium complex (hydroxymagnesium aluminate) non-absorbable, containing an amount equivalent to 29% - 40% of magnesium oxide and 18% - 26% of aluminum oxide. It reacts chemically in order to neutralize the gastric acid in the stomach, but does not have a direct effect over the production thereof. This gives rise to an increased pH of the gastric content which relieves the symptoms from hyperacidity.

It reduces la concentration of acid in the esophagus, causing an increased intraesophageal pH. It reduces pepsin activity, which contributes to the control of gastroesophageal reflux. The time of the beginning of the action thereof is intermediate and its duration is extended. The elimination via thereof is rectal and fecal. It is indicated for the treatment of hyperacidity, gastroduodenal ulcers, gastroesophageal reflux.

In order to achieve the proper antacid effect in the stomach, magaldrate should be administered between 1 and 3 hours after the meals for extending the neutralizing effect and before going to bed.

In order to neutralize about 1mEq of gastric hydrochloric acid between 15mEq and 20mEq of magaldrate are needed. Magaldrate is contraindicated en patients with severe renal dysfunction, appendicitis or appendicitis symptoms, rectal or gastrointestinal bleeding non diagnosed, ulcerative colitis, diverticulitis, colostomy, ileostomy, intestinal obstruction, hypoparathyroidism and sarcoidosis.

The chronic use of magaldrate can lead to a systemic alkalosis. The adverse reactions from the use of magaldrate in patients with chronic renal dysfunction cause changes in the state of mind or mental state (neurotoxicity), osteomalacia and osteoporosis, anorexia, muscular weakness, unusual weight loss, unusual fatigue or weakness. Magaldrate may alter the action of many medicaments so it is important to separate 2 hours the intake of magaldrate from any other medicament. Under a pH between 3 and 5, magaldrate traps 100% of the pepsin, which makes proteolysis inactive and favors the healing of the lesions from the mucous membrane. Magaldrate has a capacity to bind bile acids and lysolecithin similar to cholestyramine, which reduces the aggressiveness from the duodenogastric reflux.

### OBJECTS OF THE INVENTION

The main object of the present invention is to provide a composition of medicament for the treatment of gastric disorders, which reduces the dosage of each element in order to substantively avoid or reduce side effects.

Another object is to achieve a composition for the treatment of gastric problems which, in spite of a reduction in the concentration of the components thereof, is able to achieve a therapeutic effect at least equal to the effects from those of the art state.

Other objects and advantages of the present invention would be apparent from the study of the following description and accompanying drawings with illustrative purposes only rather than limitative.

### BRIEF DESCRIPTION OF THE INVENTION

With the aim of suppressing the drawbacks which the compositions from the art state present to address the gastric problems from the acid-peptic disease and symptoms related to the excessive production of gastric acid in addition to contributing to the cicatrization, prevention of recurrences of symptoms and complications, the development of a pharmaceutical composition which protection thereof is intended through the present application was carried out, composed by the combination of type-2 histamine-receptor-inhibiting agent, a polymer (inert silicone) and a hydroxymagnesium aluminate complex in a single dosage unit.

The present invention describes a pharmaceutical composition made-up of a combination of H₂-receptor inhibitors such as Famotidine or Ranitidine; a methylated siloxane lineal polymer (inert silicone) such as Dimeticone or Simeticone and an antacid such as aluminum, magnesium or calcium salts, e.g., a hydroxymagnesium aluminate complex such as Magaldrate, which is administered for the prevention and/or treatment of the acid-peptic disease and symptoms related to the excessive production of gastric acid in addition to contributing to the cicatrization, prevention of recurrences of symptoms and complications. Such composition can be formulated as a solution, suspension, gel, tablet, capsule or syrup.

The combination of the active principles mentioned above causes a synergic effect when they are jointly administered than when they are independently administered, providing benefits such as: Fewer doses, greater speed of action and fewer side effects.

### DETAILED DESCRIPTION OF THE INVENTION

The present invention is applied to the pharmaceutical industry field and has the aim of offering a treatment for the acid-peptic disease directed to avoid the external factors which could negatively influence on the evolution of said disease through the administration of a pharmaceutical composition which fulfill the object of relieving the manifested symptoms, promote ulcer cicatrization as well as preventing recurrences of symptomatic esophageal erosions or ulcers and complications manifested during the suffering of the acid-peptic disease.

The development of the pharmaceutical composition, object of the present invention, was carried out by combining different active principles with specific therapeutic activity, among which there is a type-2 histamine-receptor-inhibiting agent known as Famotidine; an antiflatulence agent known as Dimeticone or Simeticone and an antacid agent such as Magaldrate, which produce a synergic effect when they are jointly administered in a single dosage unit than when they are independently administered, providing benefits such as: fewer dosages, greater speed of action and fewer adverse effects in patients suffering from the acid-peptic disease.

In order to perform the development of the pharmaceutical composition mentioned above, different trials were conducted by individually administering each one of the active principles Famotidine, Dimeticone and Magaldrate with the aim to assess the behavior thereof, and the combination of said active principles in a single dosage unit was later performed, being thus noted that there fewer doses were administered and there was a greater speed of action and minimum side effects during the treatment of patients with acid-peptic disease.

Shortly, the present invention describes a pharmaceutical composition made-up of a combination of H₂-receptor inhibitors such as Famotidine or Ranitidine; a methylated siloxane lineal polymer (inert silicone) such as Dimeticone or Simeticone and an antacid agent such as any of aluminum, magnesium or calcium salt, e.g., a hydroxymagnesium aluminate complex such as Magaldrate, which is administered for the prevention and/or treatment of the acid-peptic disease and symptoms related to the excessive production of gastric acid in addition to contributing to the cicatrization, prevention of recurrences of symptoms and complications.

During the multiple trials it could be determined the alkalinity from famotidine and the excellent results from using a microcapsulated famotidine could be determined.

The invention has been described sufficiently such that a person with middle knowledge in the art is able to reproduce and obtain the results mentioned herein in the present invention. However, any person skilled in the art concerned with the present invention can be able to make modifications not described in the present application; nonetheless, if for the application of such modifications in a certain composition, the subject-matter claimed in the following claims is required, said structures shall be comprised within the scope of the invention.

Having thus described the present invention, it is considered as a novelty and, therefore, it is related as property that contained in the following claims:

## Claims

1. Synergic combination for the prevention and/or treatment of the acid-peptic disease and symptoms related to the excessive production of gastric acid **characterized in that** it comprises a type-2 histamine-receptor-inhibiting agent; an antiflatulence agent, an antacid agent and pharmaceutically acceptable excipients.

2. Synergic combination for the prevention and/or treatment of the acid-peptic disease and symptoms related to the excessive production of gastric acid in accordance to previous claim, **characterized in that** the formulated type-2 histamine-receptor-inhibiting agent is the active principle Famotidine.

3. Synergic combination for the prevention and/or treatment of the acid-peptic disease in accordance to claims 1 or 2, **characterized in that** the formulated antiflatulence agent is the active principle is Dimeticone or Simeticone.

4. Synergic combination for the prevention and/or treatment of the acid-peptic disease in accordance to any of claims 1 to 3, **characterized in that** the formulated antacid agent is the active principle Magaldrate.

5. Synergic combination for the prevention and/or treatment of the acid-peptic disease in accordance to any of claims 1 to 4, **characterized in that** the formulated antacid agent can be sodium bicarbonate or any aluminum, magnesium or calcium salt.

6. Synergic combination for the prevention and/or treatment of the acid-peptic disease in accordance to any of claims 1 to 5, **characterized in that** the formulated antacid agent can be an of the group consisting of: Aluminum Hydroxide, Magnesium Hydroxide, Calcium Carbonate, Aluminum-Magnesium metasilicate, Aluminum-Magnesium dimetasilicate, Aluminum Phosphate, Hydrated Magnesium, Magnesium Mesotrisilicate or Magnesium Trisilicate.

7. Synergic combination for the prevention and/or treatment of the acid-peptic disease in accordance to any of claims 2 to 6, **characterized in that** the active principle Famotidine is present in the formulation in a concentration range from 0.12 to 112 mg per dosage unit.

8. Synergic combination for the prevention and/or treatment of the acid-peptic disease in accordance to any of claims 2 to 6, **characterized in that** the active principle Famotidine is present in a concentration of 10.0 mg per dosage unit.

9. Synergic combination for the prevention and/or treatment of the acid-peptic disease in accordance to any of claims 3 to 8, **characterized in that** the active principle Dimeticone or Simeticone is present in the formulation in a concentration range from 10.0 to 1000 mg per dosage unit.

10. Synergic combination for the prevention and/or treatment of the acid-peptic disease in accordance to any of claims 3 to 8, **characterized in that** the active principle Dimeticone or Simeticone is present in the formulation in a concentration of 100 mg per dosage unit.

11. Synergic combination for the prevention and/or treatment of the acid-peptic disease in accordance to any of claims 3 to 10, **characterized in that** the active principle Magaldrate is present in the formulation in a concentration range from 545 to 2192 mg per dosage unit.

12. Synergic combination for the prevention and/or treatment of the acid-peptic disease in accordance to any of claims 3 to 10, **characterized in that** the active principle Magaldrate is present in the formulation in a concentration of 800 mg per dosage unit.

13. Synergic combination for the prevention and/or treatment of the acid-peptic disease in accordance to claims 2, 3, and 4, **characterized in that** Famotidine is present in a percentage from 0.001% to 1%, preferably from 0.01% to 0.15%.

14. Synergic combination for the prevention and/or treatment of the acid-peptic disease in accordance to claim 11, **characterized in that** Famotidine is present in a percentage of 0.09%.

15. Synergic combination for the prevention and/or treatment of the acid-peptic disease in accordance to claims 2, 3, and 4, **characterized in that** Famotidine or Simeticone is present in a percentage from 0.1% to 10%, preferably from 0.5% to 1.5%.

16. Synergic combination for the prevention and/or treatment of the acid-peptic disease in accordance to claim 13, **characterized in that** Dimeticone or Simeticone is present in a percentage of 0.9%.

17. Synergic combination for the prevention and/or treatment of the acid-peptic disease in accordance to claims 2, 3, and 4, **characterized in that** Magaldrate is present in a percentage from 5.0% to 20%, preferably from 6% to 10%.

18. Synergic combination for the prevention and/or treatment of the acid-peptic disease in accordance to claim 15, **characterized in that** Magaldrate is present in a percentage of 7.3%.

19. Synergic combination for the prevention and/or treatment of the acid-peptic disease in accordance to claim 1, **characterized in that** the combination is formulated in a single dosage unit for oral administration in the form of suspension.

20. Synergic combination for the prevention and/or treatment of the acid-peptic disease in accordance to claim in any of claims 2 to 19, **characterized in that** Famotidine is microencapsulated.
